# FASCICULE DE BREVET EUROPEEN

(11) **EP 2 991 548 B1**
(45) Date de publication et mention de la délivrance du brevet: **01.01.2020**
(21) Numéro de dépôt: 14718684.5
(22) Date de dépôt: 26.03.2014
(51) Int. Cl.: A61B 5/087, A61B 5/097

(54) **SYSTÈME ET DISPOSITIF POUR LA MESURE D'UN DÉBIT D'UN FLUX D'AIR EXPIRÉ OU INSPIRÉ**
SYSTEM UND VORRICHTUNG ZUR MESSUNG DER STRÖMUNGSGESCHWINDIGKEIT VON EXSPIRATORISCHEM ODER INHALIERTEM LUFTSTROM
SYSTEM AND DEVICE FOR MEASURING THE RATE OF FLOW OF AN EXHALED OR INHALED AIRFLOW

(30) Priorité: 30.04.2013 FR 1353974
(43) Date de publication de la demande: 09.03.2016
(73) Titulaire: FIM Medical, 69100 Villeurbanne (FR)
(72) Inventeur: HANNES, Benjamin, F-69630 Chaponost (FR)
(74) Mandataire: Verriest, Philippe
(86) Numéro de dépôt international: PCT/FR2014/050718
(87) Numéro de publication internationale: WO 2014/177781

(56) Documents cités:
- FR-A1- 2 016 180
- US-A1- 2002 029 003

## Description

La présente invention concerne un dispositif et un système pour la mesure d'un débit d'un flux d'air expiré ou inspiré par une personne. L'invention concerne également un procédé de fabrication du dispositif.

Il est connu d'utiliser un pneumotachographe pour la mesure d'un débit d'un flux d'air expiré ou inspiré par une personne. Le pneumotachographe comprend un conduit tubulaire dans lequel la personne expire ou inspire. Un tel dispositif est par exemple décrit dans le document FR 2 016 180 A1.

Le conduit tubulaire est pourvu d'une structure interne destinée à créer une perte de charge en obstruant partiellement le conduit tubulaire.

Le pneumotachographe comprend également un appareil de mesure de pression raccordé au conduit tubulaire pour mesurer une pression différentielle de part et d'autre de la structure ou à deux emplacements distants de la structure dans le sens d'écoulement du flux.

Le capteur de Fleisch est un pneumotachographe dans lequel la structure présente une pluralité de capillaires parallèles au sens d'écoulement. En traversant la structure, le flux d'air se sépare en une pluralité d'écoulements de type Poiseuille, chaque écoulement traversant un capillaire.

Il est connu de mesurer une pression différentielle entre deux emplacements distants d'un même capillaire. La distance séparant les deux emplacements et la nature de l'écoulement de type Poiseuille permettent de déduire le débit du flux d'air.

Il est connu de disposer d'une structure réalisée à partir d'un feuillard en inox crénelé superposé à un feuillard plat. Le feuillard crénelé et le feuillard plat sont enroulés pour créer la structure comprenant une pluralité de capillaires.

Cette disposition donne satisfaction en ce que la structure présente une pluralité de capillaires parallèles au sens d'écoulement du flux d'air permettant de séparer le flux en une pluralité d'écoulements de type Poiseuille.

Toutefois, le procédé d'obtention de la structure à partir d'un feuillard crénelé et d'un feuillard plat est complexe à mettre en œuvre. Les tolérances géométriques des dimensions des capillaires doivent être respectées. Les disparités dimensionnelles ont un effet sur la pression différentielle mesurée et sont susceptibles d'induire une erreur de mesure, c'est pourquoi ce type de dispositif nécessite une calibration.

De plus, le coût de fabrication impose une utilisation du même dispositif pour plusieurs personnes, ce qui peut engendrer un risque de contamination microbienne d'une personne à l'autre si les procédures d'utilisation et de décontaminations sont mal respectées.

La présente invention vise à résoudre tout ou partie des inconvénients mentionnés ci-dessus.

A cet effet, la présente invention concerne un dispositif pour la mesure d'un débit d'un flux d'air expiré ou inspiré par une personne tel que défini par la revendication 1.

La pluralité de canaux engendre une perte de charge imposée au flux traversant la structure. La première et la deuxième gorge sont des ouvertures agencées pour la mesure d'une pression différentielle en deux emplacements distants de la structure dans le sens d'écoulement du flux d'air.

La mesure de la pression différentielle associée à la connaissance de la géométrie des canaux permet de déduire le débit du flux d'air traversant le conduit tubulaire.

De préférence, la pluralité de canaux est dimensionnée pour réaliser une partition du flux d'air en une pluralité d'écoulements de type Poiseuille.

Le dispositif selon l'invention constitue un élément à usage unique pouvant notamment être réalisé en une seule pièce par injection. L'intérêt de réaliser le dispositif en une seule pièce par injection est d'obtenir une pièce avec des aspects dimensionnels scrupuleusement identiques. Les dispositifs sont donc interchangeables et le système ne nécessite plus de calibration.

L'intérêt de réaliser la mesure de la pression différentielle sur une pluralité de canaux est d'augmenter l'exactitude de la mesure. En effet, la pression différentielle mesurée est la moyenne des pressions différentielles de la pluralité de canaux périphériques.

Selon un aspect de l'invention, les canaux de la pluralité de canaux périphériques présentent des dimensions identiques.

Selon un aspect de l'invention, le conduit tubulaire et la structure sont monobloc.

Selon un aspect de l'invention, la structure s'étend sur la section du conduit tubulaire.

Le conduit tubulaire comprend une première portion libre et une deuxième portion libre disposées de part et d'autre de la structure.

Selon un aspect de l'invention, la première et la deuxième gorge sont distantes par rapport aux extrémités des canaux périphériques.

Les écoulements des canaux sont ainsi établis entre la première et la deuxième gorge. Ainsi chaque écoulement présente, sur les sections entre la première et la deuxième gorge, un profil transversal de vitesses d'écoulement constant avec un profil d'écoulement non turbulent.

Selon un aspect de l'invention, le conduit tubulaire est un conduit de révolution, l'axe de révolution étant confondu avec la direction d'extension du conduit tubulaire.

Selon un aspect de l'invention, la première circonférence est comprise dans un premier plan orthogonal à l'axe de révolution et dans lequel la deuxième circonférence est comprise dans un deuxième plan orthogonal à l'axe de révolution.

Selon un aspect de l'invention, les canaux présentent, dans tout plan orthogonal à l'axe de révolution entre les extrémités des canaux, des sections identiques.

Selon un aspect de l'invention, les canaux sont régulièrement répartis autour d'un canal central.

Selon un aspect de l'invention, le conduit tubulaire est un tube droit.

De préférence, le diamètre de la face intérieure du conduit tubulaire est constant.

De préférence, le dispositif comprend une embouchure tubulaire.

L'embouchure est destinée à être positionnée en partie dans la bouche d'un utilisateur du dispositif lors de la mesure du débit du flux d'air expiré ou inspiré par cet utilisateur.

De préférence, l'embouchure tubulaire présente une direction d'extension confondue avec l'axe de révolution du conduit tubulaire. L'embouchure tubulaire présente des sections orthogonales par rapport à l'axe de révolution de forme circulaire et/ou ovoïde.

De préférence, l'embouchure tubulaire présente des avancées vers l'extérieur de l'embouchure tubulaire. Chaque avancée s'étend selon un contour extérieur partiel ou total de l'embouchure tubulaire, le contour appartenant à un plan orthogonal à l'axe de révolution.

De préférence, le conduit tubulaire présente une collerette s'étendant vers l'extérieur du conduit tubulaire. De préférence, la collerette est orthogonale à l'axe de révolution.

De préférence, le conduit tubulaire, la structure, l'embouchure tubulaire et la collerette sont monobloc.

Selon un aspect de l'invention, la face extérieure comprend trois surfaces, chacune agencée pour être une surface de portée de joint, la première surface et la deuxième surface étant ménagées de part et d'autre de la deuxième gorge ; la deuxième surface et la troisième surface étant ménagées de part et d'autre de la première gorge.

Selon un aspect de l'invention, la première surface est disposée à une première distance de l'axe de révolution, la seconde surface est disposée à une seconde distance de l'axe de révolution et la troisième surface est disposée à une troisième distance de l'axe de révolution de sorte que la première distance soit inférieure à la seconde distance et que la seconde distance soit inférieure à la troisième distance.

De préférence lesdites surfaces sont circulaires, chacune étant agencée pour être une surface de portée d'un joint à lèvre. Cette disposition permet de disposer d'un emplacement de mesure de pression dans la première gorge et dans la deuxième gorge en apposant des joints sur lesdites surfaces.

Les dispositions permettent d'assurer une insertion facilitée d'un dispositif selon l'invention dans un appareil de mesure de pression selon un axe d'insertion correspondant à l'axe de révolution du dispositif, de façon à coopérer avec des joints disposés de façon coaxiale et décalée selon l'axe d'insertion, les joints présentant des dimensions internes décroissantes dans la direction d'insertion.

La présente invention concerne également un système comprenant un dispositif tel que décrit ci-dessus, un appareil de mesure de pression agencé pour mesurer simultanément au moins deux pressions au niveau de deux emplacements pourvus de prises de raccordement, et au moins un raccord étanche prévu entre l'appareil et le dispositif. Le raccord étanche est fixé sur l'appareil de mesure de manière à mettre en communication fluidique chaque gorge avec une prise de raccordement définie.

De préférence, le dispositif et l'appareil de mesure sont solidarisés de manière amovible.

De préférence, l'appareil de mesure comprend une ouverture pour l'introduction du dispositif dans l'appareil de mesure. L'ouverture est agencée pour que le dispositif s'introduise dans l'appareil de mesure selon l'axe de révolution, la deuxième extrémité du dispositif étant la première à s'introduire dans l'appareil de mesure.

De préférence, la collerette présente une étendue radiale supérieure à l'ouverture de l'appareil de mesure. La collerette est agencée pour venir en butée contre la surface extérieure de l'appareil de mesure. Ladite butée est agencée pour le positionnement du dispositif par rapport à l'appareil de mesure.

La première et la deuxième gorge du dispositif sont à l'intérieur de l'appareil de mesure lorsque le dispositif est solidarisé à l'appareil de mesure.

De préférence, l'appareil de mesure comprend un système d'encliquetage coopérant avec la collerette du dispositif pour la solidarisation du dispositif à l'appareil de mesure.

De préférence, l'appareil de mesure comprend un poussoir à ressort et une gâchette agencée pour actionner le système d'encliquetage. Lorsque le système d'encliquetage est manipulé à l'aide de la gâchette, le poussoir à ressort, agencé pour exercer une force sur la collerette, désolidarise le dispositif de l'appareil de mesure et éjecte le dispositif hors de l'appareil de mesure.

Cette disposition permet d'éjecter le dispositif de l'appareil après utilisation sans avoir à intervenir manuellement sur le dispositif. Ainsi, un second utilisateur ne touche pas le dispositif utilisé précédemment par un premier utilisateur. La contamination entre le premier et le deuxième utilisateur ou toute autre personne manipulant le système est limitée.

De préférence, trois joints à lèvres circulaires sont rapportés sur la paroi interne de l'ouverture de l'appareil de mesure et sont agencés pour coopérer respectivement avec la première surface, la deuxième surface et la troisième surface.

Cette disposition permet d'établir une communication fluidique étanche entre la première gorge et une première prise de raccordement d'une part et la deuxième gorge et une deuxième prise de raccordement de l'appareil de mesure d'autre part.

La présente invention concerne également un procédé de fabrication d'un dispositif selon l'invention par injection, dans lequel l'injection de la matière est réalisée au cours de la même étape d'injection.

De toute façon l'invention sera bien comprise à l'aide de la description qui suit en référence au dessin schématique annexé représentant, à titre d'exemple non limitatif, une forme d'exécution de ce système et de ce dispositif.
Figure 1 est une vue en perspective d'un dispositif pour la mesure d'un débit d'un flux d'air expiré ou inspiré par une personne selon un premier mode de réalisation.
Figure 2 est une vue de face du dispositif selon le premier mode de réalisation.
Figure 3 est une vue de côté du dispositif selon le premier mode de réalisation.
Figure 4 est une coupe transversale du dispositif selon le premier mode de réalisation.
Figure 5 est une vue en perspective du dispositif selon un second mode de réalisation.
Figure 6 est une vue en perspective du dispositif selon un troisième mode de réalisation.
Figure 7 est une vue de face du dispositif selon le deuxième mode de réalisation.
Figure 8 est une vue de face du dispositif selon le troisième mode de réalisation.
Figure 9 est une vue de côté du dispositif selon le deuxième mode de réalisation.
Figure 10 est une vue de côté du dispositif selon le troisième mode de réalisation.
Figure 11 est une vue d'un système comprenant le dispositif et un appareil de mesure selon le deuxième mode de réalisation.

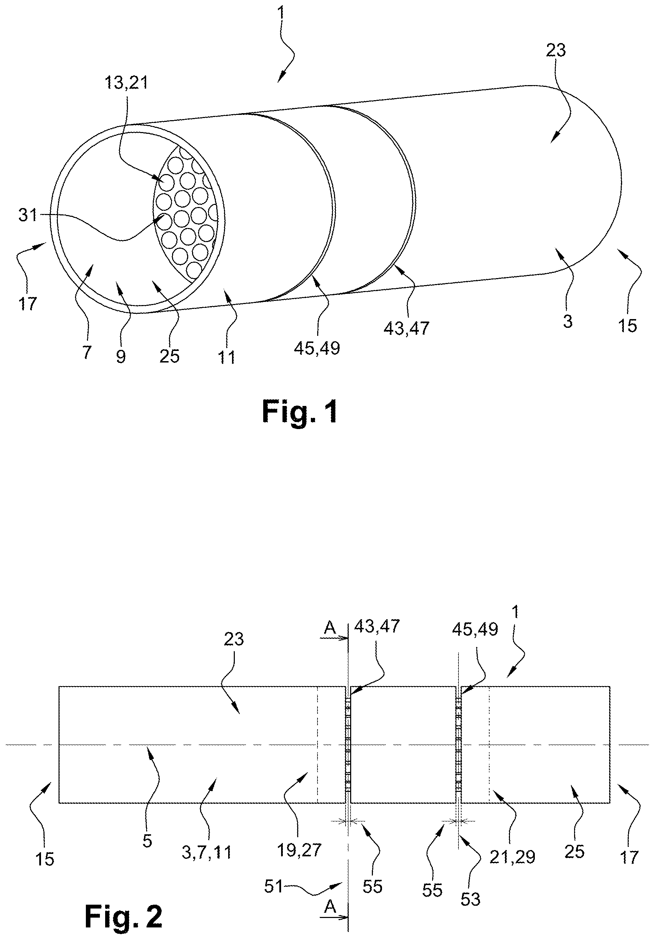

Comme illustré aux figures 1 et 2, selon un premier mode de réalisation, un dispositif 1, pour la mesure d'un débit d'un flux d'air expiré ou inspiré par une personne, comprend un conduit tubulaire 3. Le conduit tubulaire 3 est un conduit de révolution dont l'axe de révolution 5 est une droite. Le conduit tubulaire 3 est cylindrique et comprend une paroi 7 présentant une face intérieure 9 et une face extérieure 11.

Le dispositif 1 comprend également une structure 13 disposée à l'intérieur du conduit tubulaire 3 à distance d'une première 15 et d'une deuxième 17 extrémité du conduit tubulaire 3.

La structure 13 présente une première extrémité 19 et une deuxième extrémité 21 selon la direction d'extension du conduit tubulaire 3. Une première portion libre 23 du conduit tubulaire 3 est située entre la première extrémité 15 du conduit tubulaire 3 et la première extrémité 19 de la structure 13. De même, une deuxième portion libre 25 est située entre la deuxième extrémité 21 de la structure 13 et la deuxième extrémité 17 du conduit tubulaire 3.

La première extrémité 19 de la structure 13 est comprise dans un premier plan transversal 27 à l'axe de révolution 5 et la deuxième extrémité 21 de la structure 13 est comprise dans un deuxième plan transversal 29 à l'axe de révolution 5.

La structure 13 est solidaire de la face intérieure 9 de la paroi 7 du conduit tubulaire 3 et s'étend sur la section du conduit tubulaire 3 entre la première 19 et la deuxième 21 extrémité de la structure 13.

La structure 13 présente une pluralité de canaux 31 ou capillaires s'étendant parallèlement à l'axe de révolution 5 du conduit tubulaire 3 entre la première extrémité 19 de la structure 13 et la deuxième extrémité 21 de la structure 13. La pluralité de canaux 31 met en communication fluidique l'intérieur de la première portion libre 23 du conduit tubulaire 3 et l'intérieur de la deuxième portion libre 25 du conduit tubulaire 3.

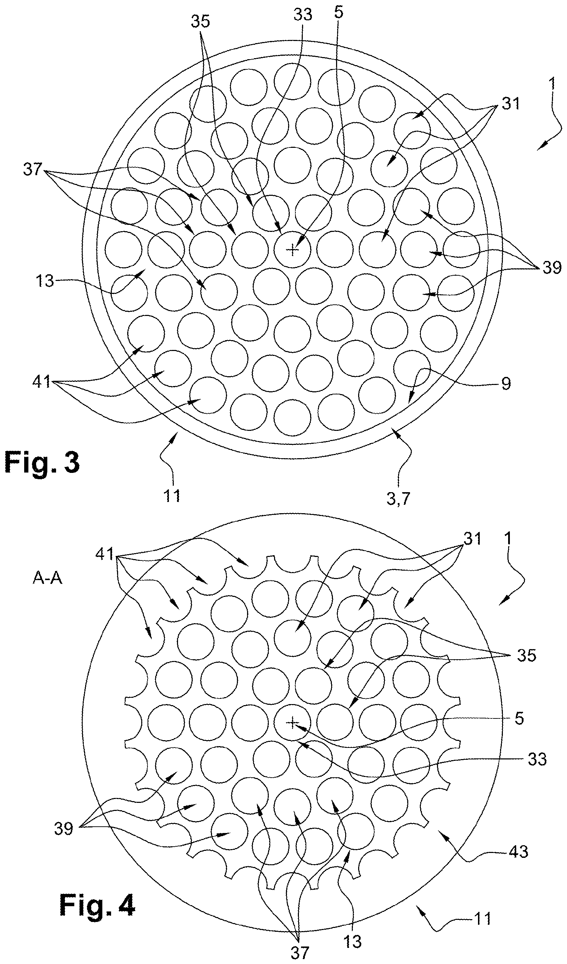

Comme illustré à la figure 3, les canaux 31 présentent une section circulaire de diamètre identique. Les canaux 31 sont régulièrement répartis autour d'un canal central 33. L'axe d'extension du canal central est confondu avec l'axe de révolution 5 du conduit tubulaire 3.

Un premier groupe 35 de canaux 31 entoure le canal central 33, les axes d'extension des canaux 31 du premier groupe 35 étant équidistants de l'axe du canal central 33.

Un deuxième groupe 37 de canaux 31 entoure les canaux du premier groupe 35, les axes d'extension des canaux 31 du deuxième groupe 37 étant équidistants de l'axe du canal central 33.

Un troisième groupe 39 de canaux 31 entoure les canaux du deuxième groupe 37, les axes d'extension des canaux 31 du troisième groupe 39 étant équidistants de l'axe du canal central 33.

Un quatrième groupe 41 de canaux 31 entoure les canaux du troisième groupe 39, les axes d'extension des canaux 31 du quatrième groupe 39 étant équidistants de l'axe du canal central 33.

Les distances radiales, par rapport à l'axe de révolution 5 du conduit tubulaire 3, séparant le canal central 33 et les canaux du premier groupe 35 sont identiques aux distances radiales séparant les canaux du premier 35 et second 37 groupe, aux distances radiales séparant les canaux du second 37 et troisième 39 groupe, et aux distances radiales séparant les canaux du troisième 39 et quatrième 41 groupe.

Chaque canal 31 appartenant à un groupe 35, 37, 39, 41 est situé à égale distance des canaux attenants appartenant au même groupe 35, 37, 39, 41.

Les canaux 31 du quatrième groupe 41 constituent les canaux périphériques 41 de la structure 13.

Comme illustré à la figure 2, le dispositif 1 présente une première gorge 43 et une deuxième gorge 45. Chaque gorge 43, 45 débouche sur la face extérieure 11 de la paroi 7 du conduit tubulaire 3 et présente une communication fluidique avec les canaux périphériques 41 de la structure 13. La deuxième gorge 45 est ménagée à distance de la première gorge 43.

La première gorge 43 s'étend sur une première circonférence 47 de la face extérieure 11 du dispositif 1, la première circonférence 47 étant comprise dans un premier plan orthogonal 51 à l'axe de révolution 5 du conduit tubulaire 3.

La deuxième gorge 45 s'étend sur une seconde circonférence 49 de la face extérieure 11 du dispositif 1, la seconde circonférence 49 étant comprise dans un deuxième plan orthogonal 53 à l'axe de révolution 5 du conduit tubulaire 3.

Il est à noter que le premier 51 et le deuxième 53 plan orthogonal sont situés entre le premier 27 et le deuxième 29 plan transversal. Ainsi tout écoulement circulant dans un canal 31 est établi entre le premier 51 et le deuxième 53 plan orthogonal, c'est-à-dire que l'écoulement présente un profil transversal de vitesse d'écoulement constant avec un régime d'écoulement non turbulent sur toutes les sections dudit canal entre le premier 51 et le deuxième 53 plan orthogonal.

La première 43 et la deuxième gorge 45 présentent une largeur 55 identique et constante, la largeur 55 étant la dimension des gorges 43, 45 selon l'axe de révolution 5 du conduit tubulaire 3.

Comme illustré à la figure 4, une coupe A-A selon le premier plan orthogonal 51 laisse apparaitre les ouvertures des canaux périphériques 41 au niveau de la première gorge 43.

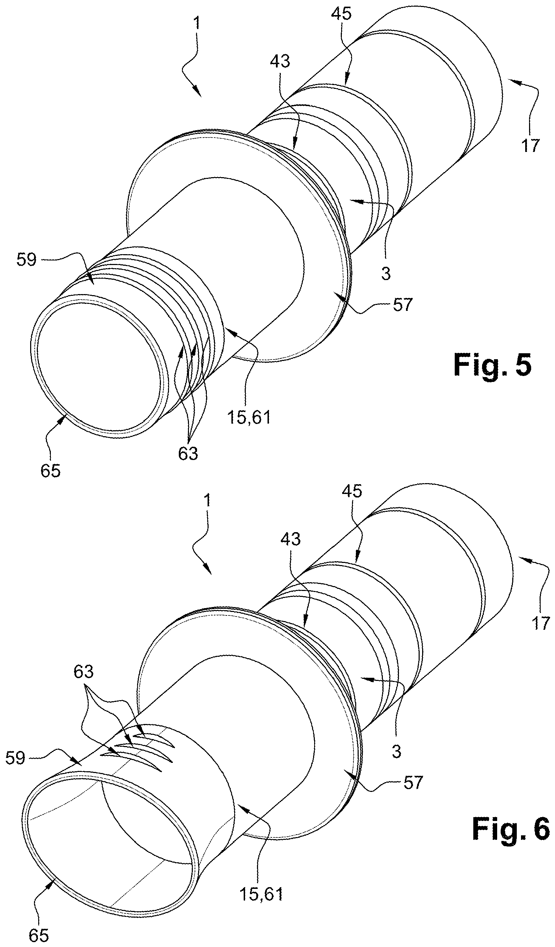

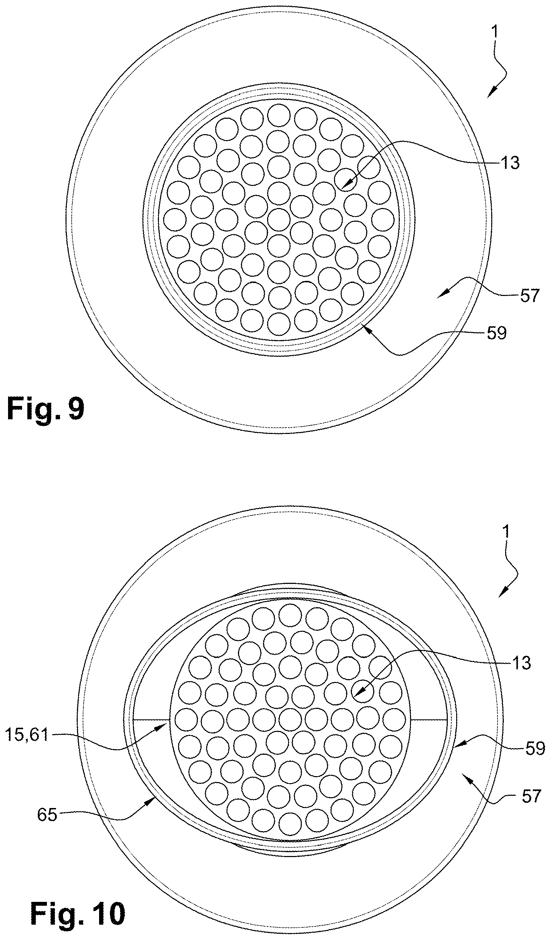

Comme illustré aux figures 5 à 10, selon un deuxième et un troisième mode de réalisation, le dispositif 1 comprend en outre une collerette 57. La collerette 57 est rapportée sur la face extérieure 11 du conduit tubulaire 3 et s'étend selon une direction orthogonale à l'axe de révolution 5.

Une embouchure tubulaire 59 est rapportée sur la première extrémité 15 du conduit tubulaire 3 de manière étanche. L'embouchure tubulaire 59 présente une première extrémité 61 de section identique à la section de la première extrémité 15 du conduit tubulaire 3.

L'embouchure tubulaire 59 présente des avancées 63 orientées vers l'extérieur de l'embouchure tubulaire 59. Chaque avancée 63 s'étend selon un contour extérieur total ou partiel de l'embouchure tubulaire 59, le contour appartenant à un plan orthogonal à l'axe de révolution 5.

Selon un aspect de l'invention, le conduit tubulaire, la structure, l'embouchure tubulaire et la collerette sont monobloc et réalisés au cours d'une même étape d'injection.

Selon le second mode de réalisation, illustré aux figures 5, 7 et 9, l'embouchure tubulaire 59 est un tube dont l'axe est l'axe de révolution 5. Chaque avancée 63 s'étend sur une circonférence de l'embouchure tubulaire 59.

Selon le troisième mode de réalisation, illustré aux figures 6, 8 et 10, l'embouchure tubulaire 59 s'étend selon l'axe de révolution et présente une seconde extrémité 65 de section ovoïde. L'embouchure 61 présente un changement de section progressif entre la première extrémité 61, de section circulaire, et la deuxième extrémité 65, de section ovoïde. Chaque avancé 63 s'étend sur une portion d'une circonférence de l'embouchure tubulaire 59 et est symétrique à une autre avancée 63 par rapport au point d'intersection du plan comprenant ladite circonférence et l'axe de révolution 5.

Ci-dessus, nous avons décrit le dispositif 1 pour la mesure d'un débit d'air selon trois modes de réalisation.

Le dispositif 1 est compris dans un système 67 pour la mesure d'un débit d'un flux d'air expiré ou inspiré par une personne. Il est entendu que la personne est considérée comme l'utilisateur du dispositif et du système.

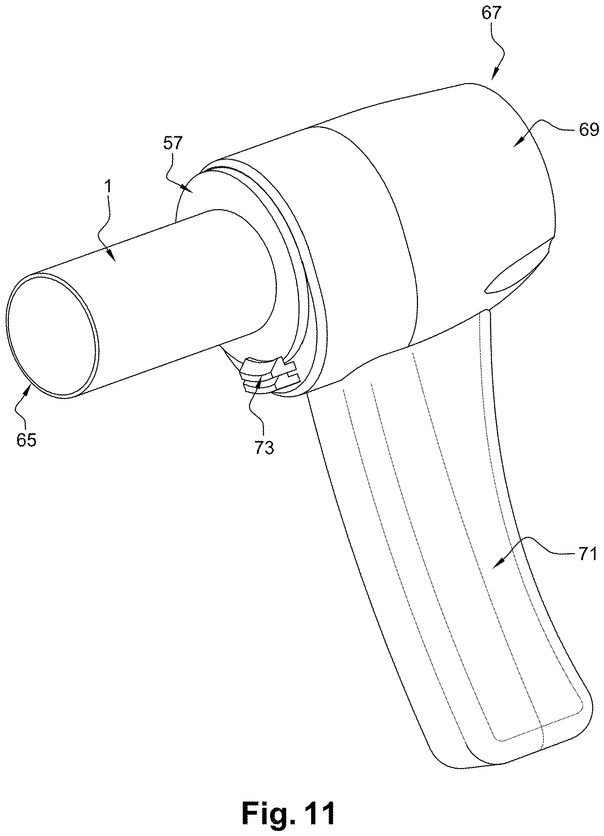

Comme illustré à la figure 11, selon le deuxième mode de réalisation, le système 67 comprend un appareil de mesure 69 de pression agencé pour mesurer simultanément au moins deux pressions au niveau de deux emplacements de l'appareil 69 pourvus de prises de raccordement.

L'appareil comprend une poignée de préhension 71 agencée pour positionner la première extrémité du dispositif dans la bouche de l'utilisateur du système 67.

Le dispositif 1 et l'appareil de mesure 69 sont solidarisés de manière amovible. Pour ce faire, l'appareil de mesure 69 comprend une ouverture destinée à l'introduction du dispositif.

Le dispositif 1 s'introduit dans l'appareil de mesure selon l'axe de révolution 5, La deuxième extrémité 17 du dispositif 1 étant la première à s'introduire dans l'appareil de mesure.

La collerette 57 est agencée pour venir en butée contre la surface extérieure de l'appareil de mesure 69 pour le positionnement du dispositif 1 par rapport à l'appareil de mesure 69.

La première 43 et la deuxième 45 gorge du dispositif sont à l'intérieur de l'appareil de mesure 69 lorsque le dispositif 1 est solidaire de l'appareil de mesure 69, comme illustré à la figure 11.

L'appareil de mesure 69 comprend un système d'encliquetage 73 coopérant avec la collerette 57 du dispositif 1 lors de la solidarisation du dispositif 1 à l'appareil de mesure 69.

Selon un aspect de l'invention, l'appareil de mesure comprend un poussoir à ressort et une gâchette agencée pour actionner le système d'encliquetage 73. Lorsque le système d'encliquetage 73 est manipulé à l'aide de la gâchette, le poussoir à ressort, agencé pour exercer une force sur la collerette 57, désolidarise le dispositif 1 de l'appareil de mesure 69 et éjecte le dispositif 1 hors de l'appareil de mesure 69.

Le système 67 comprend également des raccords étanches entre l'appareil de mesure 69 et le dispositif 1 de manière à mettre en communication fluidique chaque gorge 43, 45 du dispositif 1 avec une prise de raccordement de l'appareil de mesure 69.

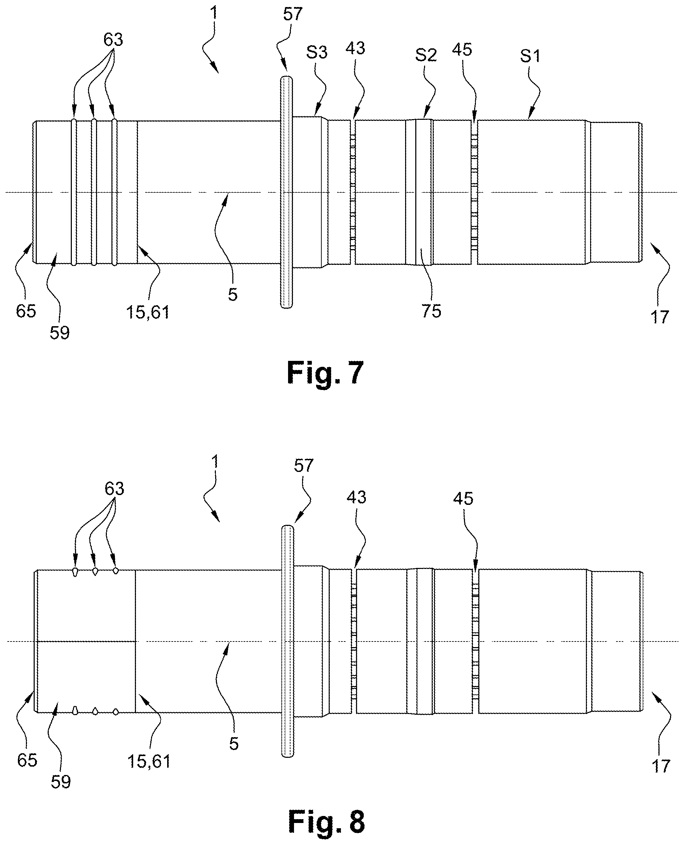

Comme illustré aux figures 7 et 8, la face extérieure 11 présente une section circulaire de diamètres différents entre la collerette 57 et la seconde extrémité 17.

En partant de la première extrémité 17 et en direction de la collerette 57, la face extérieure 11 comprend une première surface S1 circulaire entre la deuxième extrémité 17 et la deuxième gorge 45, une deuxième surface S2 circulaire entre la première gorge 43 et la deuxième gorge 45, la deuxième surface S2 étant ménagée au sommet d'un bourrelet 75. La face extérieure 11 comprend en outre une troisième surface S3 circulaire ménagée entre la première gorge 43 et la collerette 57.

La section de la troisième surface S3 est supérieure à la section de la deuxième surface S2, cette section étant elle-même supérieure à la section de la première surface S1.

Cette disposition permet l'introduction du dispositif 1 dans l'appareil de mesure 69, comme illustré à la figure 11.

L'appareil de mesure 69 comprend en outre trois joints à lèvres circulaires respectivement agencés pour être portés par les surfaces S1, S2 et S3 (non représentés à la figure 11 car ils sont disposés à l'intérieur de l'appareil de mesure 69). Les joints disposés sur les surfaces S3 et S2 assurent une communication fluidique étanche de la première gorge 43 avec une prise de raccordement. Il en est de même pour les joints disposés sur les surfaces S2 et S1 pour la deuxième gorge.

Ainsi il apparait que l'insertion du dispositif 1 dans l'appareil de mesure 69 est aisée et que l'étanchéité pour les mesures de pression est assurée de manière correcte après un simple encliquetage du dispositif 1 dans l'appareil de mesure 69.Après avoir décrit les éléments constitutifs du dispositif 1 et de l'appareil de mesure 69, nous allons détailler le fonctionnement du système 67.

Dans un premier temps, le dispositif 1 est inséré dans l'appareil de mesure 69 à travers l'ouverture de l'appareil de mesure 69. Le dispositif 1 est solidarisé à l'appareil par le système d'encliquetage 73.

Dans un deuxième temps, l'utilisateur du système insère la première extrémité 15 du conduit tubulaire 3, selon le premier mode de réalisation, ou la seconde extrémité 65 de l'embouchure tubulaire 59, selon le deuxième et troisième mode de réalisation, dans sa bouche de manière à ce que le flux expiré ou inspiré passe entièrement par le conduit tubulaire 3.

Dans un troisième temps, la personne inspire ou expire et on mesure simultanément la pression différentielle entre la première 43 et la deuxième 45 gorge avec l'appareil de mesure.

Dans un quatrième temps, on détermine le débit inspiré ou expiré à partir de la pression différentielle. En effet, les écoulements des canaux sont des écoulements de type Poiseuille. Il est alors possible d'obtenir le débit du flux d'air car pour ce type d'écoulement le débit est lié à la pression différentielle, à la section des canaux 31 et à la distance séparant le premier 51 et le deuxième 53 plan orthogonal.

Comme il va de soi, l'invention ne se limite pas à la seule forme d'exécution de ce système et de ce dispositif, décrite ci-dessus à titre d'exemple.

## Revendications

1. Dispositif (1) pour la mesure d'un débit d'un flux d'air expiré ou inspiré par une personne, comprenant :
- un conduit tubulaire (3) prévu pour conduire ledit flux d'air, le conduit tubulaire (3) comprenant une paroi (7) présentant une face intérieure (9) et une face extérieure (11), et
- une structure (13) disposée dans le conduit tubulaire (3) et comprenant une pluralité de canaux (31) destinés à réaliser une partition du flux d'air ;
**caractérisé en ce que** dispositif (1) présente :
- une première gorge (43) s'étendant le long d'une première circonférence (47) de la face extérieure (11), débouchant sur la face extérieure (11) de la paroi (7) du conduit tubulaire (3) et présentant une communication fluidique avec l'ensemble des canaux (41) situés en périphérie de la structure (13), et
- une deuxième gorge (45) s'étendant le long d'une deuxième circonférence (49) de la face extérieure (11), débouchant sur la face extérieure (11) de la paroi (7) du conduit tubulaire (3) et présentant une communication fluidique avec ledit ensemble des canaux (41) situés en périphérie de la structure (13),
la deuxième gorge (45) étant ménagée à distance de la première gorge (43).

2. Dispositif (1) selon la revendication 1, dans lequel le conduit tubulaire (3) et la structure (13) sont monobloc.

3. Dispositif (1) selon l'une des revendications 1 ou 2 dans lequel la structure (13) s'étend sur la section du conduit tubulaire (3).

4. Dispositif (1) selon l'une des revendications 1 à 3, dans lequel la première (43) et la deuxième gorge (45) sont distantes par rapport aux extrémités des canaux périphériques (41).

5. Dispositif (1) selon l'une des revendications 1 à 4, dans lequel le conduit tubulaire (3) est un conduit de révolution, l'axe de révolution (5) étant confondu avec la direction d'extension du conduit tubulaire (3).

6. Dispositif (1) selon la revendication 5, dans lequel la première circonférence (47) est comprise dans un premier plan orthogonal (51) à l'axe de révolution (5) et dans lequel la deuxième circonférence (49) est comprise dans un deuxième plan orthogonal (53) à l'axe de révolution (5).

7. Dispositif (1) selon l'une des revendications 5 ou 6, dans lequel les canaux (31) présentent, dans tout plan orthogonal à l'axe de révolution (5) entre les extrémités des canaux (31), des sections identiques.

8. Dispositif (1) selon la revendication 7, dans lequel les canaux (31) sont régulièrement répartis autour d'un canal central (33).

9. Dispositif (1) selon l'une des revendications 1 à 8, dans lequel le conduit tubulaire (3) est un tube droit.

10. Dispositif (1) selon l'une des revendications 1 à 9, dans lequel la face extérieure (11) comprend trois surfaces (S1, S2 et S3) chacune agencée pour être une surface de portée de joint, la première surface S1 et la deuxième surface S2 étant ménagées de part et d'autre de la deuxième gorge (45) ; la deuxième surface (S2) et la troisième surface(S3) étant ménagées de part et d'autre de la première gorge (43).

11. Dispositif (1) selon les revendications 10 et 5, dans lequel la première surface (S1) est disposée à une première distance de l'axe de révolution (5), la seconde surface (S2) est disposée à une seconde distance de l'axe de révolution (5) et la troisième surface (S3) est disposée à une troisième distance de l'axe de révolution (5) de sorte que la première distance soit inférieure à la seconde distance et que la seconde distance soit inférieure à la troisième distance.

12. Système comprenant :
- un dispositif (1) selon l'une des revendications 1 à 11,
- un appareil de mesure de pression agencé pour mesurer simultanément au moins deux pressions au niveau de deux emplacements pourvus de prises de raccordement, et
- au moins un raccord étanche prévu entre l'appareil et le dispositif (1),
le raccord étanche étant fixé sur l'appareil de mesure de manière à mettre en communication fluidique chaque gorge (43, 45) avec une prise de raccordement définie.

13. Procédé de fabrication d'un dispositif (1) selon l'une des revendications 1 à 11, par injection, dans lequel l'injection de la matière est réalisée au cours de la même étape d'injection.

## Patentansprüche

1. Vorrichtung (1) zur Messung einer Strömungsgeschwindigkeit von einem von einer Person ausgeatmeten oder eingeatmeten Luftstrom, umfassend:
- eine rohrförmige Leitung (3), die vorgesehen ist, um den Luftstrom zu leiten, wobei die rohrförmige Leitung (3) eine Wand (7) umfasst, die eine innere Seite (9) und eine äußere Seite (11) aufweist, und
- eine Struktur (13), die in der rohrförmigen Leitung (3) angeordnet ist, und umfassend eine Vielzahl von Kanälen (31), die ausgelegt sind, um eine Teilung des Luftstroms durchzuführen,
**dadurch gekennzeichnet, dass** die Vorrichtung (1) Folgendes aufweist:
- eine erste Auskehlung (43), die sich entlang eines ersten Umfangs (47) der äußeren Seite (11) erstreckt, die auf der äußeren Seite (11) der Wand (7) der rohrförmigen Leitung (3) mündet und eine fluidische Kommunikation mit der Gesamtheit der Kanäle (41) aufweist, die sich am Umfang der Struktur (13) befindet, und
- eine zweite Auskehlung (45), die sich entlang eines zweiten Umfangs (49) der äußeren Seite (11) erstreckt, die auf der äußeren Seite (11) der Wand (7) der rohrförmigen Leitung (3) mündet und eine fluidische Kommunikation mit der Gesamtheit der Kanäle (41) aufweist, die sich am Umfang der Struktur (13) befindet,
wobei die zweite Auskehlung (45) in einem Abstand von der ersten Auskehlung (43) angebracht ist.

2. Vorrichtung (1) nach Anspruch 1, wobei die rohrförmige Leitung (3) und die Struktur (13) aus einem Stück sind.

3. Vorrichtung (1) nach einem der Ansprüche 1 oder 2, wobei sich die Struktur (13) auf dem Abschnitt der rohrförmigen Leitung (3) erstreckt.

4. Vorrichtung (1) nach einem der Ansprüche 1 bis 3, wobei die erste (43) und die zweite Auskehlung (45) entfernt mit Bezug auf die Enden der Umfangskanäle (41) sind.

5. Vorrichtung (1) nach einem der Ansprüche 1 bis 4, wobei die rohrförmige Leitung (3) eine Drehleitung ist, wobei die Drehachse (5) mit der Ausdehnungsrichtung der rohrförmigen Leitung (3) übereinstimmt.

6. Vorrichtung (1) nach Anspruch 5, wobei der erste Umfang (47) in einer ersten orthogonalen Ebene (51) zur Drehachse (5) enthalten ist und wobei der zweite Umfang (49) in einer zweiten orthogonalen Ebene (53) zur Drehachse (5) enthalten ist.

7. Vorrichtung (1) nach einem der Ansprüche 5 oder 6, wobei die Kanäle (31) auf der gesamten orthogonalen Ebene zur Drehachse (5) zwischen den Enden der Kanäle (31) identische Abschnitte aufweisen.

8. Vorrichtung (1) nach Anspruch 7, wobei die Kanäle (31) regelmäßig um einen zentralen Kanal (33) angeordnet sind.

9. Vorrichtung (1) nach einem der Ansprüche 1 bis 8, wobei die rohrförmige Leitung (3) ein gerades Rohr ist.

10. Vorrichtung (1) nach einem der Ansprüche 1 bis 9, wobei die äußere Seite (11) drei Flächen (S1, S2 und S3) umfasst, die jeweils angeordnet sind, um eine Verbindungstragefläche zu sein, wobei die erste Fläche S1 und die zweite Fläche S2 auf beiden Seiten der zweiten Auskehlung (45) angebracht sind, wobei die zweite Fläche (S2) und die dritte Fläche (S3) auf beiden Seiten der ersten Auskehlung (43) angebracht sind.

11. Vorrichtung (1) nach Anspruch 10 und 5, wobei die erste Fläche (S1) in einem ersten Abstand von der Drehachse (5) angeordnet ist, die zweite Fläche (S2) in einem zweiten Abstand von der Drehachse (5) angeordnet ist und die dritte Fläche (S3) in einem dritten Abstand von der Drehachse (5) angeordnet ist, so dass der erste Abstand kleiner als der zweite Abstand ist, und dass der zweite Abstand kleiner als der dritte Abstand ist.

12. System, umfassend:
- eine Vorrichtung (1) nach einem der Ansprüche 1 bis 11,
- ein Gerät zur Messung des Drucks, das eingerichtet ist, um gleichzeitig mindestens zwei Drücke im Bereich von zwei Stellen zu messen, die mit Anschlussklemmen ausgestattet sind, und
- mindestens einen dichten Anschluss, der zwischen dem Gerät und der Vorrichtung (1) vorgesehen ist,
wobei der dichte Anschluss auf dem Gerät zur Messung fixiert ist, um jede Auskehlung (43, 45) mit einer definierten Anschlussklemme in fluidische Kommunikation zu bringen.

13. Verfahren zur Herstellung einer Vorrichtung (1) nach einem der Ansprüche 1 bis 11 durch Injektion, wobei die Injektion des Materials im Laufe des gleichen Schritts des Injizierens durchgeführt wird.

## Claims

1. A device (1) for measuring a flow rate of an air flow exhaled or inhaled by a person, comprising:
- A tubular conduit (3) intended to conduct said air flow, the tubular conduit (3) comprising a wall (7) having an internal face (9) and an external face (11), and
- a structure (13) disposed in the tubular conduit (3) and comprising a plurality of channels (31) intended to achieve a partition of the airflow;
**characterized in that** the device (1) has:
- a first groove (43) extending along a first circumference (47) of the external face (11), opening onto the external face (11) of the wall (7) of the tubular conduit (3) and having a fluidic communication with the set of channels (41) located at the periphery of the structure (13), and
- a second groove (45) extending along a second circumference (49) of the external face (11), opening onto the external face (11) of the wall (7) of the tubular conduit (3) and having a fluidic communication with said set of channels (41) located at the periphery of the structure (13),
the second groove (45) being formed at a distance from the first groove (43).

2. The device (1) according to claim 1, wherein the tubular conduit (3) and the structure (13) are integrally made in one-piece.

3. The device (1) according to any of claims 1 or 2, wherein the structure (13) extends over the section of the tubular conduit (3).

4. The device (1) according to any of claims 1 to 3, wherein the first (43) and the second (45) grooves are distant from the ends of the peripheral channels (41).

5. The device (1) according to any of claims 1 to 4, wherein the tubular conduit (3) is a conduit of revolution, the axis of revolution (5) being coincident with the direction of extension of the tubular conduit (3).

6. The device (1) according to claim 5, wherein the first circumference (47) is comprised within a first plane (51) orthogonal to the axis of revolution (5) and wherein the second circumference (49) is comprised within a second plane (53) orthogonal to the axis of revolution (5).

7. The device (1) according to any of claims 5 or 6, wherein the channels (31) have, in every plane orthogonal to the axis of revolution (5) between the ends of the channels (31), identical sections.

8. The device (1) according to claim 7, wherein the channels (31) are evenly distributed around a central channel (33).

9. The device (1) according to any of claims 1 to 8, wherein the tubular conduit (3) is a straight tube.

10. The device (1) according to any of claims 1 to 9, wherein the external face (11) comprises three surfaces (S1, S2 and S3) each arranged to be a seal bearing surface, the first surface S1 and the second surface S2 being formed on either side of the second groove (45); the second surface (S2) and the third surface (S3) being formed on either side of the first groove (43).

11. The device (1) according to claims 10 and 5, wherein the first surface (S1) is disposed at a first distance from the axis of revolution (5), the second surface (S2) is disposed at a second distance from the axis of revolution (5) and the third surface (S3) is disposed at a third distance from the axis of revolution (5) so that the first distance is smaller than the second distance and that the second distance is smaller than the third distance.

12. A system comprising:
- a device (1) according to any of claims 1 to 11,
- a pressure measuring apparatus arranged to simultaneously measure at least two pressures at the level of two locations provided with connection sockets, and
- at least one sealed fitting provided between the apparatus and the device (1),
the sealed fitting being fastened on the measuring apparatus so as to put each groove (43, 45) in fluidic communication with a defined connection socket.

13. A method for manufacturing a device (1) according to any of claims 1 to 11, by injection, wherein the injection of the material is carried out during the same injection step.
